# EUROPEAN PATENT SPECIFICATION

(11) **EP 2 124 973 B1**
(45) Date of publication and mention of the grant of the patent: **27.05.2015**
(21) Application number: 08718009.7
(22) Date of filing: 19.03.2008
(51) Int. Cl.: A61K 33/34, A61P 27/02, A61K 31/30, A61K 31/355, A61K 31/375, A23L 1/302, A23L 1/304, A23L 1/00, A23L 1/30, A61K 9/48, A61K 47/48, B82Y 5/00

(54) **COMPOSITION COMPRISING OMEGA-3-FATTY ACIDS AND A MASKED OR COATED COPPER SALT**
ZUSAMMENSETZUNG ENTHALTEND OMEGA-3-FETTSÄUREN UND EIN MASKIERTES ODER ÜBERZOGENES KUPFERSALZ
COMPOSITION COMPRENANT DES ACIDES GRAS OMÉGA-3 ET UN SEL DE CUIVRE MASQUÉ OU ENROBÉ

(30) Priority: 23.03.2007 EP 07104792; 28.06.2007 EP 07111322
(43) Date of publication of application: 02.12.2009
(73) Proprietor: Novartis AG, 4056 Basel (CH)
(72) Inventor: KIS, Georg Ludwig, CH-8273 Triboltingen (CH); VANDERMANDER, Jacques, F-06800 Cagnes-sur-Mer (FR)
(74) Representative: Teipel, Stephan
(86) International application number: PCT/EP2008/053283
(87) International publication number: WO 2008/116806

(56) References cited:
- US-A- 3 743 712
- US-A- 5 298 237
- US-A1- 2003 104 043
- US-A1- 2005 249 821
- US-A1- 2006 008 535
- BOSE P K ET AL: "Evidence for covalent binding between copper ions and cyclodextrin cavity: a vibrational circular dichroism study" CARBOHYDRATE RESEARCH, ELSEVIER SCIENTIFIC PUBLISHING COMPANY. AMSTERDAM, NL, vol. 323, no. 1-4, 12 January 1999 (1999-01-12), pages 63-72, XP004186501 ISSN: 0008-6215
- VOIGT RUDOLF: "Lehrbuch der pharmazeutischen Technologie" 1984, VERLAG CHEMIE , WEINHEIM 5 , XP002434798 page 234

## Description

The present invention relates to a pharmaceutical composition comprising copper-sensitive omega-3-fatty acids.

US 2003/0104043 discloses a basic therapeutic unit for topical application to the skin for the alleviation of spider veiins comprising a copper gluconate and a carrier, which may be a liposome.

US 5,298,237 discloses a gel composition for the prevention of treatment of gingivitis consisting of a gel carrier, e.g. gelatin and ascorbic acid and copper sulfate. The ascorbic acid and the copper sulfate are kept in separate gel carriers to prevent interaction.

US 2005/0249821 discloses a nutritional supplement composition for the treatment of retinal diseases such as macular degeneration comprising vitamin C, A, E, zinc, copper, omega-3-fatty acids (EPA and DHA) and taurine.

Omega-3-fatty acids and a number of vitamins and other pharmaceutically effective compounds or compositions are susceptible to decomposition when in contact with copper salts. This decomposition is primarily a problem during storage of such compounds and/or compositions and in particular when water is present. Said presence of water applies also to minute amounts of water, such as humidity per se, traces of water in one of the components of a composition and/or of a compound, or from environmental humidity.

There are a number of compositions, which contain a copper salt as an important and hence often mandatory ingredient. In such a situation, the interaction of a copper salt and the copper sensitive compound is controlled by the provision(s) provided in the next paragraphs, i.e. decomposition of the copper sensitive compound is substantially inhibited or typically prevented.

In a first aspect, the problem is solved by providing the copper salt with a proper masking or coating. This masking or coating typically suppresses an interaction with said copper sensitive compound, i.e. omega-3-fatty acids. The copper salt is for example masked or coated with-or embedded in-gelatin or liposomes and/or both such as for example a coated copper salt is embedded in gelatin. There are other ways of protecting copper salts, e.g. by encapsulating it with mono- and di-glycerides, as for example known from the product Descote^{®} which is commercially available.

Copper salts can form a stable complex with an appropriate cyclodextrin compound, which would typically release its free copper salt at a later stage, e.g. when deemed required e.g. upon enzymatic resolution of the complex in the gut.

Copper salts might be coated with a zwitterionic phospholipid including but not limited to phosphatidylcholine, phosphatidylserine, phosphatidylethanolamine, spingomyelin and other ceramides, as well as various other zwitterionic phospholipids.

In the enclosed examples there are a number of copper sensitive compounds which decompose if the copper is not properly masked or coated. Such a copper sensitive composition may for example contain:

### Example 1

| | | |
|---|---|---|
| Ingredients | Amount | Source |
| Vitamin C | 500 mg | Calcium ascorbate dihydrate, USP |
| Vitamin E | 400 IU | Alpha tocopheryl acetate, USP |
| Zinc | 40 mg | Zinc oxide |
| Copper | 1 mg | Cupric carbonate, basic, in acc. to Merck Index |
| Total Omega 3 fatty acids | 350 mg | Omega-3 acid triglycerides Ph.Eur. 120 mg |
| (DHA : EPA ratio from 1 : 4, 1 : 3, 1 : 2, 1 : 1, 2 : 1, 3 : 1, or 4 : 1) | | |
| Lutein | 10 mg | FloraGlo 20% natural source |
| Zeaxanthin | 2 mg | from Lutein |

### Example 2

In another experiment, the copper, e.g. cupric carbonate, basic, is incorporated in the shell of a capsule which contains the below ingredients. Specifically, the excipients for making such a shell are for example:

| | |
|---|---|
| Gelatin | 175 bloom bovine |
| Glycerin | 99%, USP |
| Soybean oil flakes (hydrogenated), NF | |
| Soybean oil | USP, and |
| Colorants (if required), organic and/or inorganic, e.g. Titanium dioxide USP, black iron oxide E172 and/or red iron oxide E172 | |

The stability of the above composition is investigated in a setup where the copper is not masked, and in a situation wherein the copper is masked in the shell, and hence not in direct contact with the copper sensitive ingredients. The stability of the described masking/coating method correlates with the degree of decomposition of such a copper sensitive compound and/or composition.

### Example 3

| | |
|---|---|
| 240 mg Fish oil with 70% DHA | label 160 mg Omega 3 fatty acids |
| Docosahexaeonic acid (DHA) | label 130 mg DHA |
| Eicosapentaenoic acid (EPA) as part of fish oil | label 11 mg |
| 91.5 mg Calcium ascorbate | label 60 mg Vitamin C |
| 31.343 mg d,l Alpha-tocopheryl acetate | label 20 mg Vitamin E |
| 55 mg Lutein 20% in safflower oil | label 10 mg Lutein |
| 12.447 mg Zinc oxide | label 10 mg Zinc |
| 0.45 mg Cupric carbonate basic | label 0.25 mg Copper |
| Zeaxanthin as part of Lutein | label 0.8 mg |

The above composition may contain other excipients for the production of a targeted galenic formulation, e.g. for making a shell of a capsule or the like.

### Example 4

### Stability of Omega 3 Fatty Acids in Soft Gelatin Capsules after 2 Days' Storage at 80[deg.] C. (Ambient Humidity i.e. 50-70% Relative Humidity)

### (Change in the content of the Omega 3 fatty acid's components)

The assays to measure the percentage in weight of EPA and DHA after storage are carried out by gas chromatography by the standard method described in the European Pharmacopoeia 5.4.

| **Omega 3 fatty acids' components** | **Formulation with unmasked Copper** | **Formulations with masked Copper** | |
|---|---|---|---|
| | Formulations as disclosed in example 3: (with Cupric carbonate, basic). The shell is identical to the shell disclosed in example 2 but does not contain any Copper | Formulation as disclosed in example 3, wherein the Cupric carbonate, basic; is replaced by DESCOTE^{®} (coated Copper gluconate) 2.250 mg corresponding to 0.25 mg Copper. The shell is identical to the shell disclosed in example 2 but does not contain any Copper | Formulation as disclosed in example 3, however, the Copper being omitted from said formulation. Copper is however in the shell, namely 0.45 mg of Cupric carbonate, basic (corresponding to 0.25 mg Copper) as disclosed in example 2 |
| EPA in weight % | 5.0 % decrease | 1.7 % decrease | unchanged(no decrease) |
| DHA in weight % | No detectable change | No detectable change | unchanged (no decrease) |
| Other omega 3 fatty acids** than EPA and DHA in weight % | 1.9 % decrease | 0.4 % decrease | unchanged (no decrease) |

| | | | |
|---|---|---|---|
| **Other Omega 3 fatty acids denote the sum of alpha-linolenic acid (ALA), stearidonic acid (SA), eicosatetraenoic acid (ETA) and docosapentaenoic acid (DPA). | | | |

### Bibliographic Convention:

As used herein, copper or a copper salt are used interchangeably and pertain preferably Cu²⁺ but may also include Cu⁺. A copper salt comprises preferably a pharmaceutically acceptable anion such as chloride, oxide, hydroxide, gluconate, carbonate, sulfate or the like.

As used herein, an omega-3-fatty acid are mainly but not only eicosapentaenoic acid (EPA) and docosahexaenoic acid (DHA).

As used herein, the term masked, coated or embedded copper or copper salt pertains to a copper salt and a masking, coating or embedding agent, which effectively prevents a direct interaction of said copper salt with said copper sensitive compound and/or composition.

As used herein, a digestible capsule material is for example the shell material described in the above Example 2, but is in general any capsule or capsule shell or shell material being used in the state-of-the-art for pharmaceutical/dietary/nutraceutical capsules, typically being organic polymeric compositions and typically being pharmaceutically compatible (non-toxic, bio-degradable, digestible in the gut or stomach as deemed suitable).

As used herein, the term pharmaceutical composition typically refers to a food supplement composition and/or neutraceutical composition and vice versa.

In another aspect the present invention relates to a masked or coated copper salt in a drug delivery system for use in the treatment of age related macular degeneration (AMD) or diabetic retinopathy (DR).

The invention further relates to a storage stable pharmaceutical composition comprising an omega-3-fatty acid and a coated or masked copper salt.

The invention further relates to a method of stabilizing a pharmaceutical composition comprising one or more copper sensitive omega-3-fatty acids from copper salt dependant decomposition, comprising masking or coating the copper salt in said pharmaceutical composition.

The invention further relates to a drug delivery system comprising a digestible capsuleshell, one or more copper sensitive omega-3-fatty acids, and a copper salt characterized in that said copper salt is placed into the shell material of said drug delivery system.

The digestive capsule material may comprise in the fill the following ingredient vitamin C which comprises or substantially consists of calcium ascorbate, vitamin E which comprises or substantially consists of d,l alpha tocopheryl acetate, zinc which comprises or substantially consists of zinc oxide, copper which comprises or substantially consists of Copper oxide, Copper sulfate, Copper carbonate or Copper gluconate, and an omega 3 fatty acid which comprises or substantially consists of Omega-3 acid triglycerides Ph.Eur.

## Claims

1. A pharmaceutical composition comprising one or more copper sensitive omega-3-fatty acids and a masked or coated copper salt to stabilize the pharmaceutical composition from copper salt dependent decomposition.

2. The composition in accordance to claim 1 wherein said copper is coated with gelatin, a liposome, or a mono- and di-glyceride.

3. The composition in accordance to any of the preceding claims wherein said copper is copper gluconate, copper oxide, copper carbonate or copper chloride.

4. The composition in accordance to any of the preceding claims wherein said coating is a zwitterionic phospholipid including but not limited to phosphatidylcholine, phosphatidylserine, phosphatidylethanolamine, spingomyelin and other ceramides, as well as various other zwitterionic phospholipids.

5. The composition in accordance to any of the preceding claims wherein said copper is a copper cyclodextrin complex.

6. A drug delivery system comprising a digestible capsule shell and a fill, wherein said fill comprises one or more copper-sensitive omega-3-fatty acids and said shell is formed from digestible capsule material comprising a copper salt, **characterized in that** said copper salt is placed into the shell material such that said copper salt is masked and therefore not in contact with said copper-sensitive compounds in said fill.

7. The drug delivery system of claim 6, wherein said fill comprises the following ingredients: vitamin C which comprises calcium ascorbate, vitamin E which comprises d,l alpha tocopheryl acetate, zinc which comprises zinc oxide and an omega 3 fatty acid which comprises Omega-3 acid triglycerides.

8. The drug delivery system of claim 6 for use in the treatment and/or prevention of age-related macular degeneration (AMD) and/or diabetic retinopathy (DR).

9. The drug delivery system of claim 6, wherein said digestible capsule material comprises a digestible capsule material that is digestible in the gastrointestinal tract.

10. A method of stabilizing a pharmaceutical composition comprising one or more copper sensitive omega-3-fatty acids from copper salt dependant decomposition, comprising masking or coating the copper salt in said pharmaceutical composition.

11. The method of claim 10, wherein said copper is coated with gelatin, a liposome, or a mono- and di-glyceride.

12. The method of claim 10, wherein said copper is copper gluconate, copper oxide, copper carbonate or copper chloride.

13. The method of claim 10, wherein said coating is a zwitterionic phospholipid including but not limited to phosphatidylcholine, phosphatidylserine, phosphatidylethanolamine, spingomyelin and other ceramides, as well as various other zwitterionic phospholipids.

14. The method of claim 10, wherein said copper is a copper cyclodextrin complex.

## Patentansprüche

1. Pharmazeutische Zusammensetzung, umfassend eine oder mehrere kupferempfindliche Omega-3-Fettsäuren und ein maskiertes oder beschichtetes Kupfersalz zur Stabilisierung der pharmazeutischen Zusammensetzung vor Kupfersalz-abhängiger Zersetzung.

2. Zusammensetzung nach Anspruch 1, wobei das Kupfer mit Gelatine, einem Liposom oder einem Mono- und Diglycerid beschichtet ist.

3. Zusammensetzung nach einem der vorstehenden Ansprüche, wobei das Kupfer Kupfergluconat, Kupferoxid, Kupfercarbonat oder Kupferchlorid ist.

4. Zusammensetzung nach einem der vorstehenden Ansprüche, wobei die Beschichtung ein zwitterionisches Phospholipid, einschließlich, aber nicht beschränkt auf Phosphatidylcholin, Phosphatidylserin, Phosphatidylethanolamin, Sphingomyelin und andere Ceramide, sowie verschiedene andere zwitterionische Phospholipide ist.

5. Zusammensetzung nach einem der vorstehenden Ansprüche, wobei das Kupfer ein Kupfer-Cyclodextrin-Komplex ist.

6. Arzneimittelabgabesystem, umfassend eine verdaubare Kapselhülle und eine Füllung, wobei die Füllung eine oder mehrere kupferempfindliche Omega-3-Fettsäuren umfasst und die Hülle aus einem verdaubaren Kapselmaterial, umfassend ein Kupfersalz, gebildet ist, **dadurch gekennzeichnet, dass** das Kupfersalz in dem Hüllenmaterial so platziert wird, dass das Kupfersalz maskiert wird und daher nicht mit den kupferempfindlichen Verbindungen in der Füllung in Kontakt kommt.

7. Arzneimittelabgabesystem nach Anspruch 6, wobei die Füllung die folgenden Inhaltsstoffe umfasst: Vitamin C, welches Calciumascorbat umfasst, Vitamin E, welches d,l-alpha-Toco-pherylactat umfasst, Zink, welches Zinkoxid umfasst, und eine Omega-3-Fettsäure, welche Omega-3-Säuretriglyceride umfasst.

8. Arzneimittelabgabesystem nach Anspruch 6 zur Verwendung bei der Behandlung und/oder Vorbeugung von altersbedingter Makuladegeneration (AMD) und/oder diabetischer Retinopathie (DR).

9. Arzneimittelabgabesystem nach Anspruch 6, wobei das verdaubare Kapselmaterial ein verdaubares Kapselmaterial umfasst, das im Gastrointestinaltrakt verdaubar ist.

10. Verfahren zur Stabilisierung einer pharmazeutischen Zusammensetzung, umfassend eine oder mehrere kupferempfindliche Omega-3-Fettsäuren, vor Kupfersalz-abhängiger Zersetzung, umfassend das Maskieren oder Beschichten des Kupfersalzes in der pharmazeutischen Zusammensetzung.

11. Verfahren nach Anspruch 10, wobei das Kupfer mit Gelatine, einem Liposom oder einem Mono- und Diglycerid beschichtet wird.

12. Verfahren nach Anspruch 10, wobei das Kupfer Kupfergluconat, Kupferoxid, Kupfercarbonat oder Kupferchlorid ist.

13. Verfahren nach Anspruch 10, wobei die Beschichtung ein zwitterionisches Phospholipid, einschließlich, aber nicht beschränkt auf Phosphatidylcholin, Phosphatidylserin, Phosphatidylethanolamin, Sphingomyelin und andere Ceramide, sowie verschiedene andere zwitterionische Phospholipide ist.

14. Verfahren nach Anspruch 10, wobei das Kupfer ein Kupfer-Cyclodextrin-Komplex ist.

## Revendications

1. Composition pharmaceutique comprenant un ou plusieurs acides gras oméga 3 sensibles au cuivre et un sel de cuivre recouvert ou masqué pour stabiliser la composition pharmaceutique de la décomposition liée au sel de cuivre.

2. Composition selon la revendication 1, dans laquelle ledit cuivre est recouvert de gélatine, d'un liposome, ou d'un mono- et di-glycéride.

3. Composition selon l'une quelconque des revendications précédentes, dans laquelle ledit cuivre est le gluconate de cuivre, l'oxyde de cuivre, le carbonate de cuivre ou le chlorure de cuivre.

4. Composition selon l'une quelconque des revendications précédentes, dans laquelle ledit revêtement est un phospholipide zwitterionique comprenant, mais non limité à la phosphatidylcholine, la phosphatidylsérine, la phosphatidyléthanolamine, la sphingomyéline et autres céramides, ainsi que divers autres phospholipides zwitterioniques.

5. Composition selon l'une quelconque des revendications précédentes, dans laquelle ledit cuivre est un complexe de cytodextrine de cuivre.

6. Système de délivrance de médicaments comprenant une enveloppe de capsule digestible et un remplissage, dans lequel ledit remplissage comprend un ou plusieurs acides gras oméga 3 sensibles au cuivre et ladite enveloppe est formée à partir d'un matériau de capsule digestible comprenant un sel de cuivre, **caractérisé en ce que** ledit sel de cuivre est placé dans le matériau d'enveloppe de sorte que ledit cuivre est masqué et n'est ainsi pas en contact avec lesdits composés sensibles au cuivre dans ledit remplissage.

7. Système de délivrance de médicaments selon la revendication 6, dans lequel ledit remplissage comprend les ingrédients suivants : de la vitamine C, laquelle comprend de l'ascorbate de calcium, de la vitamine E, laquelle comprend de l'acétate de d,l alpha tocophéryle, du zinc, lequel comprend de l'oxyde de zinc et des acides gras oméga 3, lesquels comprennent des triglycérides d'acides oméga 3.

8. Système de délivrance de médicaments selon la revendication 6 pour utilisation dans le traitement et/ou la prévention de la dégénérescence maculaire liée à l'âge (DMLA) et/ou la rétinopathie diabétique (RD).

9. Système de délivrance de médicaments selon la revendication 6, dans lequel ledit matériau de capsule digestible comprend un matériau de capsule digestible qui est digestible dans le tractus gastro-intestinal.

10. Procédé de stabilisation d'une composition pharmaceutique comprenant un ou plusieurs acides gras oméga 3 sensibles au cuivre contre la décomposition liée au sel de cuivre, comprenant le masquage ou le recouvrement du sel de cuivre dans ladite composition pharmaceutique.

11. Procédé selon la revendication 10, dans lequel ledit cuivre est recouvert de gélatine, d'un liposome, ou d'un mono- et di-glycéride.

12. Procédé selon la revendication 10, dans lequel ledit cuivre est le gluconate de cuivre, l'oxyde de cuivre, le carbonate de cuivre ou le chlorure de cuivre.

13. Procédé selon la revendication 10, dans lequel ledit revêtement est un phospholipide zwitterionique comprenant, mais non limité à la phosphatidylcholine, la phosphatidylsérine, la phosphatidyléthanolamine, la sphingomyéline et autres céramides, ainsi que divers autres phospholipides zwitterioniques.

14. Procédé selon la revendication 10, dans lequel ledit cuivre est un complexe de cytodextrine de cuivre.
